Europäisches Patentamt

European Patent Office

Office européen des brevets

(11) Publication number: **0 106 894**
**B1**

(12) **EUROPEAN PATENT SPECIFICATION**

(45) Date of publication of patent specification: **15.10.86**

(21) Application number: **83901970.0**

(22) Date of filing: **18.04.83**

(86) International application number:
**PCT/US83/00579**

(87) International publication number:
**WO 83/03903 10.11.83 Gazette 83/26**

(51) Int. Cl.⁴: **G 01 N 33/574,**
**G 01 N 33/577**

(54) **METHODS AND TEST KIT FOR DIAGNOSING CANCER IN HUMANS.**

(30) Priority: **23.04.82 US 371287**

(43) Date of publication of application:
**02.05.84 Bulletin 84/18**

(45) Publication of the grant of the patent:
**15.10.86 Bulletin 86/42**

(84) Designated Contracting States:
**FR**

(56) References cited:
**Journal of the National Cancer Institute,
Volume 54, (Number 2), pages 341-343, Issued
February 1975, Levine et al.**

**Nucleic Acids Research, Volume 5, (Number 9),
pages 3439-3455, Issued September 1978,
Milestone et al.**

**Nucleic Acids Research, Volume 7, (Number 1),
pages 193-204, Issued 1979, Vold et al.**

**Nucleic Acids Research, Volume 7, (Number 4),
pages 971-980, Issued 1979 Vold et al.**

(73) Proprietor: **SRI INTERNATIONAL**
**333 Ravenswood Avenue**
**Menlo Park, California 94025 (US)**

(72) Inventor: **VOLD, Barbara Schneider**
**Apartment 5, 200 Waverly Street**
**Menlo Park, CA 94025 (US)**

(74) Representative: **Santarelli, Marc et al**
**Cabinet Rinuy et Santarelli 14, avenue de la**
**Grande Armée**
**F-75017 Paris (FR)**

(56) References cited:
**Cancer Markers, S. Sell, editor, The HUMANA
Press, Clifton, N.J., pages 445-462 Issued 1980,
Borek, E.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### Technical Field

The invention is in the field of cancer diagnosis. More particularly it concerns a method for diagnosing cancer by determining the amount of certain modified nucleosides in urine by immunoassay.

### Background Art

Ribonucleic acids (RNAs), particularly transfer RNAs (tRNAs), contain a variety of modified nucleosides which are formed after transcription of the macromolecule. When these RNAs are degraded, the majority of modified nucleosides do not appear to be catabolized to any extent in animals or humans and are excreted in significant amounts in urine. Since tRNAs are the most highly modified class of RNAs, it has been assumed that turnover of tRNA is the source of excreted modified nucleosides. Evidence for a higher turnover rate for tRNA in tumor tissue and evidence for intracellular scavenging of tRNAs with structural abnormality have been reported. Borek, E., et al, *Cancer Res* (1977) 37:3362—3366 and Nomura, Y., *FEBS Lett* (1974) 45:223—227. Elevated amounts of certain modified nucleosides in the urine of advanced cancer patients as measured by high performance liquid chromatography (HPLC) have also been reported. Speer, J. et al, *Cancer* (1979) 44:2120—2123 and Borek, E., *Cancer Markers* (1980) Ed. Sell, S., Humana Press.

Radioimmunoassays for various modified nucleosides, including N-[9-(β-D-ribofuranosyl)purin-6-ylcarbamoyl]-L-threonine ($t^6A$), have been used to evaluate the levels of such nucleosides in bacterial tRNAs. Milstone, et al, *Nucl Acids Res* (1978) 5:3439—3455; Vold, B., *Nucl Acids Res* (1979) 7:193—204; and Vold, B. et al, *Nucl Acids Res* (1979) 7:971—980. The serum levels of $N^2,N^2$-dimethylguanosine and pseudo-ridine in cancer patients determined by radioimmunoassay have also been reported. Levine, et al, *J Natl Cancer Inst* (1975) 54:341—343.

A principal object of the present invention is to provide a reproducible, noninvasive method for diagonsing human cancer based on the levels of modified nucleosides in physiological fluids. This invention uses urine as the test physiological fluid and an immunoassay to determine the amount of modified nucleoside in the urine. Another object of the invention is to provide a method for diagnosing human cancer based on the level of N-[9-(β-D-ribofuranosyl)-purin-6-ylcarbamoyl]-L-threonine in urine. Still another object is to provide a novel monoclonal anti-$t^6A$ antibody for use in such diagnosis.

### Disclosure of the Invention

One aspect of the invention is a method for determining whether a human patient has cancer comprising:

(a) determining the amount of modified nucleoside that is normally excreted in elevated amounts by human subjects that have cancer in a sample of urine of said patient; and

(b) comparing said amount with the standared amount of modified nucleoside present in urine of normal human subjects, characterized in that the modified nucleoside is N-[9-(β-D-ribofuranosyl)-purin-6-ylcarbamoyl]-L-threonine.

Another aspect of the invention is a test kit for determining whether a human patient has cancer according to the above method, characterized in that it comprises in association:

(a) labeled N-[9-(β-D-ribofuranosyl)purin-6-ylcarbamoyl]-L-threonine; and

(b) a monoclonal antibody against N-[9-(β-D-ribofuranosyl)purin-6-ylcarbamoyl]-L-threonine.

A final aspect of the invention is a monoclonal antibody against N-[9-(β-D-ribofuranosyl)purin-6-ylcarbamoyl]-L-threonine for carrying out the above method.

### Modes for Carrying Out the Invention

The invention methods may be used to diagnose any type of cellular neoplasm, including carcinomas, myelomas, and lymphomas. Examples of cancers that have been determined to be associated with elevated levels of modified nucleosides are leukemia, lung cancer, Burkitt's lymphoma, melanoma, ovarian cancer, metastic breast cancer, Hodgkin'a disease, colon cancer, bladder cancer, and bronchogenic carcinoma.

The urine samples that are used in the invention methods may be collected randomly or over a given time period, conventionally 24 h. No significant variation in the levels of modified nucleoside have been observed relative to the sample collection regimen. One of the significant advantages of the invention relative to the prior art HPLC method is that the urine samples do not have to be treated prior to being assayed. In the HPLC technique the nucleosides are separated by affinity chromatography from the sample before being assayed. The amount of urine used in the invention methods will usually range between about 1 to about 20 μl depending upon the particular nucleoside involved.

The modified nucleosides that form the basis for the diagnosis occur in significantly elevated amounts in the urine of patients having the type of cancer being diagnosed. Among the modified nucleosides that have been reported to be excreted in elevated amounts by cancer patients are 1-methylinosine, $N^2, N^2$-dimethylguanosine, pseudourine, 1-methylguanine, 7-methylguanine, and 8-hydroxy-7-methylguanine. Applicants have found that $t^6A$ is also excreted in elevated amounts by cancer patients. $t^6A$ is a particularly interesting modified nucleoside because it occurs only in tRNA.

The amounts of these modified nucleosides in urine are determined by a quantitative immunoassay.

2

Various types of quantitative immunoassays such as competitive immunoassays, direct immunoassays and indirect immunoassays may be used. These assays involve the formation of immune complexes that include a label and the detection of such complexes via the label. In competitive assays the sample is incubated with an antibody against the modified nucleoside and a known amount of labeled modified nucleoside. Any modified nucleoside (unlabeled) in the sample competes with the labeled nucleoside for antibody. The resulting immune complexes are separated and the amount of label therein is determined. The amount of modified nucleoside is determined by comparison with the effect of standards. Direct immunoassays involve incubating the sample with a labeled antibody against the modified nucleoside and separating any immune complexes that form. The amount of label therein is determined and the amount of modified nucleoside in the sample is determined by comparison with the effect of standards. In an indirect immunoassay, such as an enzyme linked immunosorbent assay (ELISA)*, the antibody is bound to a solid phase, the sample is added, and a second labeled antibody is added. Immobilized immune complexes are detected via the label.

The particular label that is used in the quantitative immunoassays are not critical. The label should be such as to provide sensitivities down to at least about 1 to 10 pmol of modified nucleoside. Examples of labels that may be used are radiolabels such as $^{32}P$, $^{125}I$, $^3H$, and $^{14}C$, fluorescent labels such as fluorescein and its derivatives, rhodamine and its derivatives, dansyl, and umbelliferone, chemiluminescers such as luciferime and 2, 3-dihydrophthalazinediones, and enzymes, such as horseradish peroxidase, alkaline phosphatase, lysozyme, and glucose-6-phosphate dehydrogenase. The antibody or nucleoside, as the case may be, may be tagged with such labels by known methods. For instance, coupling agents such as aldehydes, carbodiimides, dimaleimide, imidates, succinimides and bis-diazotized benzidine may be used to tag the antibodies with fluorescent, chemi-luminescent, or enzyme labels.

The antibodies against the modified nucleosides that are used in these immunoassays may be polyclonal (heterologous) or monoclonal (homologous). Polyclonal antibodies may be made by immunizing host animals such as mice, rabbits, and sheep using a modified nucleoside-protein carrier conjugate as an immunogen. The immunization will typically involve repeated inoculations with the immunogen, typically at least two at about one week intervals. Such inoculation will raise an immune response against the immunogen and cause the inoculated host's immune system to produce antibodies against the modified nucleoside. Serum from the immunized host will usually be collected about three to ten days after the final booster. Immunoglobulins may be separated from the serum by ammonium sulfate precipitaiton, gel electrophoresis, dialysis, chromatography, or other conventional separation and purification techniques, if desired.

Monoclonal antibodies against the modified nucleosides may be made by the somatic cell hybridization techniques of Kohler and Milstein, *Nature* (1975) 265:495 using antibody-producing cells, eg spleen or lymphoid cells, from the immunized host animal, preferably a mouse, as one of the hybridization partners. The antibody-producing cells are hybridized (fused) with an appropriate cancer (myeloma) cell line using a fusogen such as polyethylene glycol having a MW of 1,000 to 6,000 daltons. A myeloma cell line that is sensitive to a selective medium such as HAT medium (Littlefield, *Science* (1969) 145:709—710), fuses efficiently, and will support stable high level expression and secretion of antibody by its hybridization partner is used. While myeloma cells from any species may be use, murine myeloma lines having these characteristics are available currently and are preferred. Examples of such lines are those derived from the original MOPC—21 and MPC—11 mouse tumors that are available from the Salk Institute Cell Distribution Center, PO Box 1809, San Diego, California 92112. A myeloma cell:antibody-producing cell ratio in the range of about 1:10 and about 10:1 will normally be used. The individual cell concentrations will typically be in the range of about $10^6$ to $10^8$, preferably $1 \times 10^7$ to $5 \times 10^7$, cells/ml fusion medium. Balanced salt solutions containing about 30% to 60% (w/v), preferably 35% to 50% (w/v) fusion may be used as a fusion medium. After the fusion, the cells are washed with fusogen-free medium to remove fusogen. They are then seeded and cultivated in the selective medium to eliminate unhybridized parent cells and leave only hybrids that are resistant to the selective medium and possess the immortality of the myeloma parent. The cultivation will normally take about three to five weeks.

Surviving hybridomas may be examined for production of antibody against the modified nucleoside by immunoassay. Positive hybridoma clones may be subcloned by limiting dilution techniques and grown *in vitro* or *in vivo* by known techniques. The monoclonal anti-modified nucleoside antibody secreted by the subclones may be separated from the culture medium or ascites fluid when grown *in vivo* by known techniques such as ammonium sulfate precipitation, DEAE cellulose chromatography, or affinity chromatography. Further purification of the antibodies, if desired, may be achieved by ultracentrifugation and microfiltration.

The incubations of the immunoassays will be carried out under conditions that permit reaction between the antibody and modified nucleoside. Temperature, pH and duration are the most important process conditions in the incubation. Temperatures in the range of about 5°C to 40°C, preferably about

---

* ELISA is a registered trade-mark

3

37°C, will typically be used. The pH will normally be about 6 to 9, preferably about 7, and the binding reaction will usually reach equilibrium in about 10 min to 2 days, depending upon the particular antibodies and nucleosides involved. Antibody will normally be used in excess. Immune complexes may be separated from the incubation mixture by centrifugation or other conventional techniques. Separation may not be necessary in assays such as the homogeneous enzyme immunoassay (EMIT)*.

The label detection means used in the immunoassay will depend upon the particular label involved. For instance, radiolabels may be read with scintillation counters, fluorescent labels with fluorescent microscopes (fluorometers) and enzyme labels with colorimeters that detect the magnitude of color change caused by the reaction between the enzyme and the substrate.

The basic ingredients of the test kit for carrying out the t⁶A assay of the invention are labeled t⁶A and monoclonal antibody against t⁶A. These ingredients will typically be in solution in an appropriate aqueous solvent and contained in suitable dispensers. The kit may also contain a suitable buffer, carrier protein, unlabled t⁶A for preparing standards, containers in the reagents may be used, and directions for performing the assay. The components of the kit may be packaged in a conventional manner.

The following examples further illustrate the invention methods. These examples are not intended to limit the invention in any manner.

Example 1

*Urine Samples.* 24 h urine samples were obtained from patients with various advanced malignancies, previously untreated with chemotherapy, and normal volunteers at the Cancer Center of the University of New Mexico. The urines were collected from each individual in plastic containers for 24 h and kept under refrigeration until the end of collection. The urine volume was then measured and an aliquot of urine was kept frozen until the day of analysis. The amount of creatinine for each sample was determined by the colorimetric method described in the Sigma Technical Bulletin No 555 1—12, 1977.

*Antibody Preparation.* Immunogen was prepared by conjugating t⁶A to bovine serum albumin (BSA) using the method of Erlanger and Beiser, *PNAS* (1964) 52:68—74. Antibodies were raised in rabbits, a total of 5 mg immunogen (mixed with complete Freund's adjuvant) per rabbit was introduced in three injections into the hind legs on days 0, 14, and 21. The immunized rabbits were bled on day 28. Serum was precipitated twice at 0°C with 50% saturated ammonium sulfate (SAS), pH 7.0, and then dialyzed against 0.14 M NaCl, 10 mM sodium phosphate, pH 7.0 (PBS).

*Tritiated Antigen.* Tritiated t⁶A was prepared by tritium gas substitution by Moravek Biochemicals. [³H]t⁶A, $3.11 \times 10^{10}$ Bq (0.84 Ci/mmol), was 88% pure by 2D thin layer chromatography analysis using the solvent system described by Rogg, et al, *Nucleic Acids Res* (1976) 3:285—295, and 6.9 pmol was used per assay.

*Radioimmunoassay.* The saturated ammonium sulfate radioimmunoassay (SAS—RIA) for t⁶A described by Vold, B. S., *Nucleic Acids Res* (1979) 7:193—204 was used. Assays were done in 12 × 75 mm polypropylene tubes in a total reaction volume of 300 µl with 75 mM NaCl, 0.1 M boric acid, 25 mM sodium tetraborate, pH 8.3. Normal rabbit IgG was used to adjust the total protein concentration of each assay to 600 µg. The buffer, antibody, carrier protein, [³H]t⁶A, and untreated urine were mixed well, incubated at 37°C for 1 h, then cooled on ice for 5 min. The amount of urine used was chosen such that normal urine would give inhibition values between 10% and 40%. Thus, 2 µl of urine was used in each assaay. An equal volume of ice-cold SAS was added to each tube, mixed thoroughly, and incubated at 0°C for 30 min. The tubes were then centrifuged at 1800 × g for 20 min at 4°C, and the supernatant removed by aspiration through a capillary pipette. The pellets were resuspended in 200 µl of 50% SAS in borate saline buffer, mixed, centrifuged, and drained as before. The washed pellets were then suspended in 200 µl of 1X borate saline buffer, mixed, and a 75 µl added to 5 ml of Aquasol* scintillation fluid. Radioactivity was then measured in a scintillation counter. Under the conditions used for competitive inhibition, cpm bound on the filter without inhibitor and with (or without) antiserum was 1133 (66) for t⁶A. When urine was added as a source of competitive inhibitor and the same assay repeated on four different occasions, standard deviation was ± 3.7%.

The concentration of inhibition present in each urine sample was interpolated from a graph of % trace binding for the antibody when the cognate modified nucleoside had been used as competitive inhibitor. These concentrations were then expressed in mg excreted per 24 h period and normalized to the creatinine level in each sample expressed as nmol nucleoside/µmol creatinine. Speer, et al, *Cancer* (1979) 44:20—2123.

---

* EMIT is a registered trade-mark

---

* Aquasol is a registered trade-mark

**0 106 894**

Urine samples from 8 normal patients, 4 Hodgkin's patients, 4 non-Hodgkin's lymphoma patients, 5 lung cancer patients (small cell, adenocarcinoma, squamous cell and larte cell) and 8 "other" cancer (breast, head and neck, bowel) patients were tested. None of the patients had been treated previously by chemotherapy. Assay results for the cancerous patients were compared by group with the assay results from the normal patients. Significance of variation in the levels of $t^6A$ in the samples was established using a t test comparing each cancer group to the normal group. A P value less than 0.05 was considered a significant variation. The results of these comparisons were:

| Significance of Variation (P) | | | |
|---|---|---|---|
| Lymphomas | | Solid Tumors | |
| Hodgkins | Non-Hodgkins | Lung | Other |
| Not significant | P<0.05 | P<0.001 | P<0.02 |

As these data indicate, elevated levels of $t^6A$ in urine is a clear marker for all the cancers tested except perhaps Hodgkins disease. The lack of significant variation in the Hodgkins patients may be attributable to the small number of patients involved.

Example 2

*Monoclonal Antibody Preparation and Testing.* A Balb/C mouse was immunized with the $t^6$A-BSA conjugate of Example 1 (100 µg) in complete Freund's adjuvant intraperitoneally at day 0. At day 7 the mouse was boosted with 100 µg of the conjugate in PBS intravenously. The mouse spleen was removed at day 10. Spleen cells were fused with P3/63/Ag 8.653 murine myeloma cells according to Kennet's fusion protocol (Kennet, et al, (1979) *Cell Fusion.* In Methods in Enzymology, Academic Press, New York pp 345—357). The fusogen was polyethylene glycol (1540 mw) at 38% w/v.

Media from wells containing surviving cells were tested for anti-$t^6$A antibody by radioimmunoassay. Cells from one of the positive wells (# G8) were subcloned by limiting dilution into wells containing normal mouse spleen cells as feeders. Subclones were tested for antibody production by radio-immunoassay and one, identified as G8—3, was chosen as a source of monoclonal anti-$t^6$A antibody.

Cells from clone G8—3 were then frozen, re-established in culture, and subcloned a second time *in vitro* according to the same procedure as the first sub-cloning. Two clones from the second sub-cloning were retained (#9D4 and #5C4).

The screening of the clones was done using a solid phase assay. A conjugate of $t^6$A to human serum albumin was made and used to coat wells of a microtiter plate. Culture fluid was then added followed by a second antibody which was labeled with $^{125}$I and which recognizes mous IgG.

Antibodies from the culture fluid were isolated by means of an affinity column chromatography step using Protein A-Sepharose* CL—4B. The bound fraction from this column was then tested in the saturated ammonium sulfate radioimmunoassay for $t^6$A (as described previously) in which binding of $[^3H]t^6$A was observed.

**Claims**

1. A method for determining whether a human patient has cancer comprising:

(a) determining the amount of modified nucleoside that is normally excreted in elevated amounts by human subjects that have cancer in a sample of urine of said patient; and

(b) comparing said amount with the standard amount of the modified nucleoside present in urine of normal human subjects, characterized in that the modified nucleoside is N-[9-(β-D-ribofuranosyl)purin-6-ylcarbamoyl]-L-threonine.

2. The method of claim 1 wherein the cancer is a non-Hodgkins lymphoma or a solid tumor.

3. The method of claim 1 or 2 wherein the determination of step (a) is made by a quantitative competition immunoassay in which a urine sample from the patient is incubated with an antibody against N-[9-(β-D-ribofuranosyl)purin-6-ylcarbamoyl]-L-threonine and labeled N-[9-(β-D-ribofuranosyl)purin-6-ylcarbamoyl]-L-threonine.

4. The method of claim 3 wherein the antibody is a monoclonal antibody.

5. The method of claim 3 wherein the label is tritium.

* Sepharose is a registered trade-mark

6. A test kit for determining whether a human patient has cancer according to the method of any one of claims 1 or 3, characterized in that it comprises in association:

(a)  labeled N-[9-(β-D-ribofuranosyl)purin-6-ylcarbamoyl]-L-threonine; and

(b)  a monoclonal antibody against N-[9-(β-D-ribofuranosyl)purin-6-ylcarbamoyl]-L-threonine.

7. Monoclonal antibody against N-[9-(β-D-ribofuranosyl)purin-6-ylcarbamoyl]-L-threonine for carrying out the method of any one of claims 1 or 3.

8. The monoclonal antibody of claim 7 wherein the antibody is a murine antibody of the IgG class.

**Patentansprüche**

1. Verfahren zur Diagnose von Krebs beim Menschen, wobei

(a) in einer Probe des Urins des Patienten die Menge an modifiziertem Nukleosid bestimmt wird, die normalerweise in erhöhten Mengen von Menschen, die an Krebs erkrankt sind, ausgeschieden wird; und

(b) diese Menge mit der Standardmenge des modifizierten Nukleosids verglichen wird, die im Urin von normalen Menschen vorhanden ist, dadurch gekennzeichnet, daß das modifizierte Nukleosid N-[9-(β-D-ribofuranosyl)purin-6-ylcarbamoyl]-L-threonin ist.

2. Verfahren nach Anspruch 1, wobei der Krebs ein Non-Hodgkins-Lymphom oder ein solider Tumor ist.

3. Verfahren nach Anspruch 1 oder 2, wobei die Bestimmung in Stufe (a) durchgeführt wird mittels eines quantitativen kompetitiven Immunoassays, wobei eine Urinprobe des Patienten inkubiert wird mit einem Antikörper gegen N-[9-(β-D-ribofuranosyl)purin-6-ylcarbamoyl]-L-threonin und markiertem N-[9-(β-D-ribofuranosyl)purin-6-ylcarbamoyl]-L-threonin.

4. Verfahren nach Anspruch 3, wobei der Antikörper ein monoklonaler Antikörper ist.

5. Verfahren nach Anspruch 3, wobei das Markierungselement Tritium ist.

6. Testausstattung zur Diagnose von Krebs beim Menschen nach der Methode eines der Ansprüche 1 oder 3, dadurch gekennzeichnet, daß sie in Kombination aufweist:

(a)  markiertes N-[9-(β-D-ribofuranosyl)purin-6-ylcarbamoyl]-L-threonin; und

(b)  einen monoklonalen Antikörper gegen N-[9-(β-D-ribofuranosyl)purin-6-ylcarbamoyl]-L-threonin.

7. Monoklonaler Antikörper gegen N-[9-(β-D-ribofuranosyl)purin-6-ylcarbamoyl]-L-threonin zur Durchführung des Verfahrens nach Anspruch 1 oder 3.

8. Monoklonaler Antikörper nach Anspruch 7, wobei der Antikörper ein Murin-Antikörper der IgG-Klasse ist.

**Revendications**

1. Méthode permettant de détecter si un patient humain est atteint de cancer, consistant:

(a)  à déterminer, dans un échantillon d'urine dudit patient, la quantité de nucléoside modifié qui est normalement excrété en quantités élevées par des sujets humains qui sont atteints de cancer; et

(b)  à comparer ladite quantité avec une quantité de référence du nucléoside modifié présent dans l'urine de sujets humains normaux, caractérisée en ce que le nucléoside modifié est la N-[9-(β-D-ribofurannosyl)purine-6-ylcarbamoyl]-L-thréonine.

2. Méthode suivant la revendication 1, dans laquelle le cancer est un lymphome n'appartenant pas au type Hodgkins ou une tumeur solide.

3. Méthode suivant la revendication 1 ou 2, dans laquelle la détermination dans l'étape (a) est effectuée par un immuno-essai quantitatif par compétition, dans lequel un échantillon d'urine du patient est incubé avec un anticorps contre la N-[9-(β-D-ribofurannosyl)purine-6-ylcarbamoyl]-L-thréonine et la N-[9-(β-D-ribofurannosyl)purine-ylcarbamoyl]-L-thréonine marquée.

4. Méthode suivant la revendication 3, dans laquelle l'anticorps est un anticorps monoclonal.

5. Méthode suivant la revendication 3, dans laquelle le marqueur est le tritium.

6. Kit analytique permettant de déterminer si un patient humain est atteint de cancer conformément à la méthode suivant l'une quelconque des revendications 1 ou 3, caractérisé en ce qu'il comprend en association:

(a)  de la N-[9-(β-D-ribofurannosyl)purine-6-ylcarbamoyl]-L-thréonine marquée; et

(b)  un anticorps monoclonal contre la N-[9-(β-D-ribofurannosyl)purine-6-ylcarbamoyl]-L-thréonine.

7. Anticorps monoclonal contre la N-[9-(β-D-ribofurannosyl)purine-6-ylcarbamoyl]-L-thréonine pour la conduite de la méthode suivant l'une quelconque des revendications 1 ou 3.

8. Anticorps monoclonal suivant la revendication 7, qui est un anticorps murin de la classe IgG.